Europäisches Patentamt

European Patent Office

Office européen des brevets

(11) Publication number : **0 480 639 A2**

# (12) EUROPEAN PATENT APPLICATION

(21) Application number : **91309110.4**

(22) Date of filing : **04.10.91**

(51) Int. Cl.⁵ : **A61N 1/06, A61B 17/39**

(30) Priority : **09.10.90 JP 269578/90**

(43) Date of publication of application :
**15.04.92 Bulletin 92/16**

(84) Designated Contracting States :
**BE CH DE ES FR GB IT LI LU NL SE**

(71) Applicant : **Yamada, Shiro**
**No. 2-7-1-606, Mita**
**Minato-ku, Tokyo (JP)**

(72) Inventor : **Yamada, Shiro**
**No. 2-7-1-606, Mita**
**Minato-ku, Tokyo (JP)**

(74) Representative : **Palmer, Roger et al**
**PAGE, WHITE & FARRER 54 Doughty Street**
**London WC1N 2LS (GB)**

(54) **Electrode assembly for high-frequency heating and coagulating apparatus.**

(57)    An electrode assembly suitable for use in an apparatus for heating and coagulating a living tissue by a high-frequency current, comprises a planar electrode (3) having a terminal (5), which is connected to a high-frequency power supply, and a working face (3) in the form of a flat or convex surface, and a hollow cooling chamber (4) for cooling the working face from the back side thereof, said planar electrode and hollow cooling chamber being formed integrally with each other.

Fig.1

Jouve, 18, rue Saint-Denis, 75001 PARIS

The present invention relates to an electrode assembly suitable for use in an apparatus for heating and coagulating a living tissue by a high-frequency current, and more specifically to an electrode assembly equipped with a cooling device, by which a high-frequency current is caused to flow from above the skin through a living tissue in order to selectively heat and coagulate the subcutaneous tissue. The high-frequency heating and coagulating apparatus equipped with this electrode assembly is usable for remedying superficial vascular diseases such as hemangioma, and tumors in a shallow site under the skin or for removing fat by atrophy of the subcutaneous fat without receiving any thermal injury to the human epidermis.

It has been used in surgical operations or the like for a long time to cauterize or heat and coagulate a living tissue by utilizing a high-frequency current. For example, it is described in Japanese Patent Application Laid-Open No. 211060/1987 to burn off the affected part in a living cavity. It is also described in Japanese Patent Application Laid-Open Nos. 185847/1982 and 24933/1988 to heat and coagulate a living tissue with a view toward arresting bleeding of the tissue.

Besides, it has recently been proposed by the present inventor that a needle-like working electrode is thrust into the skin to cause a high-frequency current to pass through the skin so as to heat and coagulate an intended subcutaneous tissue alone without leaving any scar on the epidermis (see Japanese Utility Model Application Laid-Open No. 114147/1984 and Japanese Patent Application Laid-Open No. 277948/1987). Namely, according to the device of Japanese Utility Model Application Laid-Open No. 114147/1984, the needle-like electrode is thrust into the skin near the affected part of the human body to cause the high-frequency current to pass through the skin, thereby heating and coagulating hypertrophied capillary vessels, by which hemangioma is caused, to extinguish them. The operation is conducted without leaving any thermal injury on the epidermis by applying an insulating coating to the surface of the needle-like electrode except its tip. Also, according to the invention of Japanese Patent Application Laid-Open No. 277948/1987, artificial hair is implanted in the skin of the human body and at the same time, a high-frequency current is caused to flow through a hair-implanting needle to heat and coagulate the subcutaneous tissue near the root part of the artificial hair implanted, thereby increasing the fixing rate of the artificial hair.

The invention of Japanese Patent Application Laid-Open No. 211060/1987 is characterized in that the electrode is brought into direct contact with the affected part to cause a heavy high-frequency current to flow through the affected part, thereby burning off the affected part. Also, in the invention of Japanese Patent Application Laid-Open No. 24933/1988, the electrode is brought into direct contact with the affected part to cause a high-frequency current to pass through the affected part, thereby heating and coagulating the affected part to effect its hemostasis or the like. In order to bring the electrode into direct contact with the affected part as described above, there is however a disadvantage that the skin must be incised to expose the affected part before using this process.

The invention of Japanese Patent Application Laid-Open No. 185847/1982 features that a high-frequency current is caused to flow through the affected part via an electrically conductive fluid, thereby heating and coagulating the affected part. In this process, no electrode is brought into direct contact with the affected part, but the fluid is brought into direct contact with the affected part. Therefore, the process involves a disadvantage that it can not be used unless a tube for feeding the fluid is inserted deep into the body cavity in order to bring the fluid into direct contact with the affected part.

Beside, according to both device of Japanese Utility Model Application Laid-Open No. 114147/1974 and invention of Japanese Patent Application Laid-Open No. 277948/1987, an electrically insulating coating film is applied to the surface of the working electrode leaving its tip alone so as not to cause the high-frequency current to flow through the surface of the skin, thereby heating and coagulating the intended subcutaneous tissue alone without leaving any scar on the surface of the skin. However, this process is accompanied by disadvantages that the needle-like working electrode must be thrust into the skin and moreover, its remedy region is extremely limited because the working electrode is in the form of a needle, so that extremely poor efficiency is only obtained when using for purposes other than the hair implantation.

According to the present invention, a subcutaneous tissue is coagulated by indirectly heating the tissue from above the skin without inserting or thrusting any electrode into the body of a patient.

Generally speaking, when coagulation by high-frequency heating is caused to undergo, a high-frequency current having a frequency of 0.5-10 MHz, preferably of 2.5 MHz or less is used. The electric current is caused to flow from a working electrode to a distributed electrode as a counter electrode with a human body interposed therebetween.

In general, when an electrode is placed on the skin to cause the high-frequency current to flow, the tissue under the skin can be coagulated by Joule heat generated by the high-frequency current. However, the skin itself is also necessarily heated, thereby causing so-called dermal burn.

It is therefore a first object of this invention to provide an electrode assembly for a high-frequency heating and coagulating apparatus, which permits selective heating and coagulation of a tissue alone in a deep site under the skin without doing any injury to the epidermis.

It is a second object of this invention to provide an electrode assembly for a high-frequency heating and coagulating apparatus, which is capable of treating an affected part positioned under the skin and extending to a relatively wide region at once without doing any injury to the epidermis.

The electrode assembly for the high-frequency heating and coagulating apparatus according to the present invention comprises a planar electrode having a terminal, which is connected to a high-frequency-current power supply, and a working face in the form of a flat or convex surface, and a hollow cooling chamber for cooling the working face from the back side thereof, said planar electrode and hollow cooling chamber being formed integrally with each other.

A high-frequency current supplied to the planar electrode having the working face in the form of the flat or convex surface flows through the skin from the working face into the body to heat the skin and the subcutaneous tissue. At that time, when a coolant is circulated through the cooling chamber provided on the back side of the planar electrode to cool the planar electrode from the inside, heat near the surface of the skin is absorbed in the electrode, so that the temperature of the skin surface is not raised. It is therefore possible to heat and coagulate the subcutaneous tissue alone in the deep site without receiving any thermal injury to both epidermis and subcutaneous tissues in the shallow site.

The forms of the planar electrode and working face are determined on the basis of the intended application and difficulty in fabrication of the electrode assembly.

It is however common to use a circular electrode and a working face shaped in a circular form correspondingly.

When the affected part is vertically elongate, a working face in the form of an oval or rectangle is however used. On the other hand, when the fat under eyes is removed, a semicircle or crescent, or if circumstances require, triangle, trapezoid or the like may be used. In any event, the shape and size according to those of the affected part will be required of the working face.

However, the peripheral part of the planar electrode is cooled to a lesser extent compared with the central part thereof. Therefore, when a planar electrode in a circular form by way of example is used, there may be instances where the surface of the skin receives a slight ring-like burn.

Accordingly, in this invention, an annular cooling surface has been provided so as to surround the periphery of the working face of the planar electrode to cool the peripheral part of the planar electrode higher, whereby the occurrence of the ring-like burn is prevented.

As a specific structure for providing such an annular cooling surface that the periphery of the working face of the planar electrode is surrounded, its purpose is attained by integrally forming the planar electrode and the hollow cooling chamber with an electrically conductive material and annularly coating the peripheral part of the planar electrode with an electrical insulating material having good thermal conductivity. Namely, the peripheral part of the planar electrode, which has been coated with the electrical insulating material, becomes impossible to give off any high-frequency current due to a coating film of the electrical insulating material. On the other hand, since the planar electrode is inherently made of a , metallic material having good thermal conductivity, and the annular coating film is also formed of the electrical insulating material having good thermal conductivity, this annular portion functions as a cooling surface. Accordingly, the region inside the annular cooling surface becomes a working face of the planar electrode.

As examples of the electrically conductive material making up the electrode, may be mentioned stainless steel, brass, phosphor bronze, silver, etc. However, brass is preferred in that it is relatively high in electrical conductivity, good in processability and cheap.

Examples of the electrical insulating material having good thermal conductivity include plastics, glass, ceramics, insulating coatings, insulating varnishes, etc. When a ceramic is used in particular, it is preferably non-porous. Namely, a ceramic material having an extremely fine particle size, for example, a sintered body or film of micronized alumina, or the like is preferred. As another embodiment for providing the annular cooling surface, its purpose is also attained by an electrode assembly in which the hollow cooling chamber is made up of an electrical insulating material having good thermal conductivity so as to have a bottom surface greater than the planer electrode, and the planar electrode is fitted in the bottom surface at the substantial center thereof, whereby a remaining portion of the bottom surface, which is outside the periphery of the electrode, is taken as an annular cooling surface.

In the case of this embodiment, a portion of the bottom surface of the cooling chamber, which corresponds to the planar electrode, may be recessed to fit the planar electrode in the recess. However, it is preferred from the viewpoint of increased cooling efficiency that only the portion corresponding to the planar electrode is hollowed out in the bottom surface of the cooling chamber to fit the planar electrode therein, whereby the planar electrode is caused to serve for a bottom surface of the cooling chamber, too.

As a further embodiment, the outer peripheral surface of a curved tube made of an electrically conductive material is coated with an electrical insulating material. The insulating film on the convex surface of the tube is then removed by grinding, thereby forming an oval working face of the planar electrode. The tube itself serves as a cooling chamber.

A coolant is introduced into the cooling chamber to cool the planar electrode from the back side thereof. Therefore, the cooling chamber is provided with an inlet port and an outlet port for the coolant.

Examples of the coolant may include liquids and gases. Water, saline, an aqueous solution of ethylene glycol or the like may be used as a liquid coolant.

Since a temperature of about 0 to -10°C is sufficient for the temperature of the coolant, water or saline may be cooled or supercooled with ice and then circulated. For that purpose, it is only necessary to construct a cooling system so as to place ice in a water tank and circulate water through the water tank by a circulating pump.

In the case of the gas, liquid nitrogen, liquid $CO_2$, Freon gas, high-pressure air or the like may be used. When each of these gases is used, its cooling system is constructed so as to provide a nozzle in the electrode assembly, jet the liquid gas or high-pressure gas out of the nozzle and adiabatically expand the gas to give heat for cooling. For that purpose, it is necessary to provide a high-pressure pipe communicating with a container for the liquid or high-pressure gas, and a nozzle adapted to jet the gas out in the cooling chamber and fitted on the tip of the pipe.

By the way, with respect to the outlet port, it is only necessary to provide several holes for discharging the gas in the outside air in optional positions of the cooling chamber except for the case where the gas requires to be recovered in particular.

When the electrode assembly according to the present invention and a distributed electrode are used as a working electrode for a high-frequency heating and coagulating apparatus and as a counter electrode thereof, respectively, a human body is placed between the working electrode and the distributed electrode, and a high-frequency current of, for example, 1 MHz and 50 W in the case where the diameter of the working electrode is 14 mm is caused to flow, the skin abutting on the working electrode is heated. When at that time, a coolant of 0°C is forcedly circulated through the cooling chamber, the skin abutting on the working electrode is cooled, so that only the subcutaneous tissue in the deep site under the working electrode is heated. When the above-described high-frequency current is caused to flow for about 3 seconds, tissues down to about 2 mm from the epidermis are not in any way changed, but deeper subcutaneous tissues down to about 6 mm are heated to about 90°C, whereby proteins in the tissues within that range are coagulated.

When at that time, the planar electrode having a working face in the form of a flat or convex surface is used, it is possible to heat and coagulate the affected part under the skin extending to a considerably wide region from above the skin, thereby attaining the intended remedy, or to coagulate and atrophy the subcutaneous adipose tissue, thereby substantially decreasing the subcutaneous fat.

However, when a relatively heavy high-frequency current is caused to flow, there may be instances where the surface of the skin receives a slight ring-like burn along the periphery of the planar electrode. This is attributed to the insufficient cooling of the planar electrode at its peripheral part.

According to other embodiments of this invention, in order to overcome the above-described defect, the cooling chamber is constructed in such a manner that the annular cooling surface provided so as to surround the periphery of the working face of the planar electrode is also cooled from its back side, whereby the peripheral part of the planar electrode is cooled higher and hence, the occurrence of the ring-like burn is prevented.

The examples of the present invention will hereinafter be described with reference to the accompanying drawings.

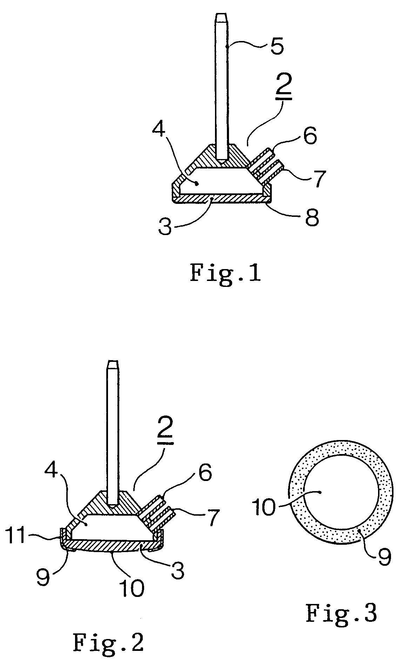

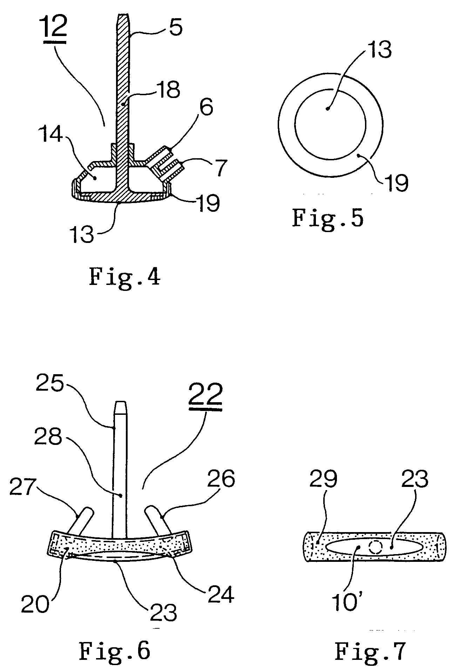

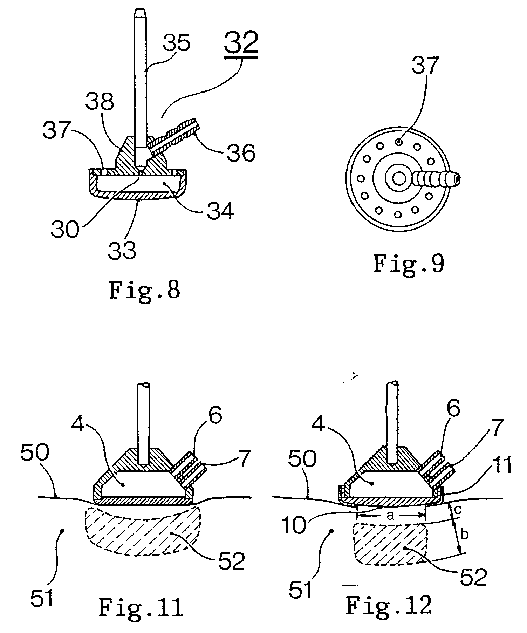

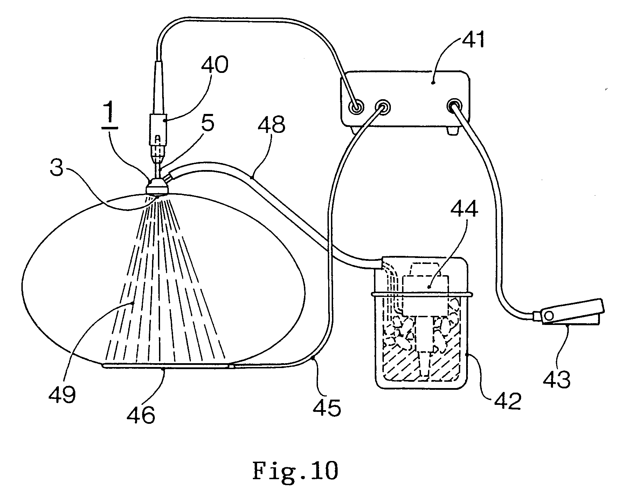

The drawings illustrate electrode assemblies for a high-frequency heating and coagulating apparatus according to embodiments of the present invention. FIG. 1 is a cross-sectional view of an electrode assembly according to an embodiment thereof; FIG. 2 is a cross-sectional view illustrating an electrode assembly according to another embodiment; FIG. 3 is a bottom plan view of the electrode assembly of FIG. 2; FIG. 4 is a cross-sectional view illustrating an electrode assembly according to a further embodiment; FIG.5 is a bottom plan view of the electrode assembly of FIG. 4; FIG. 6 is a cross-sectional view illustrating an electrode assembly according to a stillfurther embodiment; FIG. 7 is a bottom plan view of the electrode assembly of FIG. 6; FIG. 8 is a cross-sectional view illustrating an electrode assembly according to yet a still further embodiment; FIG. 9 is a bottom plan view of the electrode assembly of FIG. 8; FIG. 10 is an explanatory view illustrating a high-frequency heating and coagulating apparatus equipped with an electrode assembly according to this invention as used; FIGS. 11 and 12 are explanatory views illustrating the heated and coagulated conditions of the subcutaneous tissue as a result that the high-frequency heating and coagulating apparatus separately equipped with electrode assemblies according to this invention has been used. The numerals used in the drawings are as follows:-

1: electrode assembly;
2, 12, 22, 32: electrode bodies;
3, 13, 23, 33: planar electrodes;
4, 14, 24, 34: cooling chambers;
5: terminal; 6, 26, 36: inlet ports for coolant;

7, 27, 37: outlet port for coolant;

8: outer peripheral part;

9, 19, 29: annular cooling surfaces;

10, 10': working faces; 30: nozzle;

41: high-frequency generator;

42: coolant tank; 46: distributed electrode;

50: surface of skin; 52, 52': coagulated regions

Example 1:

In this example, an electrode assembly making use of a liquid coolant as a coolant and, in particular, corresponding to that of claim 1 will be described.

As illustrated in FIG. 1, an electrode body 2 is formed of a metal having good electrical conductivity, for example, brass, and is substantially in the form of a hollow cone as a whole. Its bottom surface serves as , planar electrode 3 and its upper conical part forms a cooling chamber 4. In the upper part of the conical cooling chamber 4, there is provided a rod-like terminal 5 for connecting to a high-frequency generator (not illustrated), and an inlet port 6 and an outlet port 7 for the liquid coolant are separately provided in its side wall.

In this drawing, the planar electrode 3 of the bottom surface is illustrated in the form of a flat surface. It is however preferred that the electrode is shaped in a slightly convex form because an outer peripheral part 8 of the planar electrode 3 is cooled to a lesser extent and hence, there may be instances where the surface of the skin receives a ring-like burn.

Example 2:

In this example, an electrode assembly corresponding to that of one of claims 3 and 4 in particular will be described. The outer periphery part of a planar electrode is coated with an electrical insulating material in order to positively prevent the occurrence of the ring-like burn in Example 1. As illustrated in FIGS. 2 and 3, an electrode body 2 is, as with that in Example 1, formed of a metal having good electrical conductivity, and is in the form of a hollow cone. Its bottom serves as a planar electrode 3 and its upper part forms a cooling chamber 4. An annular coating film 11 composed of an electrical insulating material having good thermal conductivity is applied from the outer periphery of the planar electrode 3 to the foot of the cooling chamber 4.

By this annular coating film 11, a working face 10 of the planar electrode 3 is defined to a region inside the annular coating film 11. On the other hand, the peripheral part of the planar electrode 3, which has been coated with the annular coating film 11, functions as an annular cooling surface 9.

In this example, a sintered film obtained by baking alumina powder of 500 mesh or finer and having a thickness of 150 μm is used as an electrical insulating material having good thermal conductivity.

Besides, saline cooled with ice is circulated for use as a coolant.

Example 3:

In this example, an electrode assembly corresponding to that of one of claims 3 and 6 in particular will be described.

As illustrated in FIGS. 4 and 5, an electrode body 12 comprises a planar electrode 13 made of an electrically conductive material such as a metal and a conductor 18 extending upright from an upper center of the planar electrode 13. The tip of the conductor 18 serves as a terminal 5 for connecting to a high-frequency generator (not illustrated). A cooling chamber 14 is provided so as to surround the planar electrode 13 and a lower end of the conductor 18.

The cooling chamber 14 is formed of an electrical insulating material having good thermal conductivity, for example, a plastic such as a rigid polyvinyl chloride, or a ceramic molding. An inlet port 6 and an outlet port 7 for a liquid coolant are separately provided in a side wall of the cooling chamber 14.

Since the planar electrode 13 is surrounded with the cooling chamber 14, which is a size larger than the planar electrode 3, as described above, a bottom portion of the cooling chamber 14, which is in an annular form, serves as a cooling surface 19.

Example 4:

In this example, an electrode assembly corresponding to that of one of claims 3 and 5 in particular will be described.

As illustrated in FIGS 6 and 7, an electrode body 22 is constructed by a curved tube 20 formed of an electrically conductive material such as a metal. Both ends of the tube 20 are sealed, and a conductor 28 is provided upright on an upper center thereof. The whole outer surface of the tube 20 is applied with a coating film composed of an electrical insulating material. However, a convex surface of the tube 20 is subjected to grinding to expose the metal surface, thereby forming an oval working face 10' of a planar electrode 23.

An inlet port 26 and an outlet port 27 are respectively attached on both ends of the tube, whereby the tube forms a cooling chamber 24 as a whole.

An annular region situated on the bottom surface of the cooling chamber 24 and surrounding the planar electrode 23 functions as a cooling surface 29.

By the way, the tip of the conductor 28 forms a terminal 25 for connecting to a high-frequency generator (not illustrated).

Example 5:

In this example, the case where a gaseous coolant is used as a coolant will be described.

As shown in FIGS. 8 and 9, a bottom of a hollow electrode body 32 in the form of a flattened cylinder, which is formed of a metal having good electrical conductivity, is used as a planar electrode 33, and the portion above the bottom is utilized as a cooling chamber 34.

To the upper center of the cooling chamber 34, is connected upright a high-pressure pipe 38 for introducing a coolant. An inlet port 36 for a gaseous coolant is fitted to a side wall of the high-pressure pipe 38. An upper end of the high-pressure pipe 38 is sealed by a rod-like terminal 35 for connecting to a high-frequency generator (not illustrated). An internal surface of the high-pressure pipe 38 in a joint between the high-pressure pipe 38 and the cooling chamber 34 is constricted, thereby forming a nozzle 30. A coolant, such as a liquid gas, introduced through the inlet port 36 for the gaseous coolant performs a cooling action in the following manner. It is jetted out of the nozzle 30 of the high-pressure pipe 38 to adiabatically expand and hence generate heat for cooling, thereby taking up heat from the circumference of the cooling chamber 34.

The coolant vaporized is discharged in the outside air through discharge ports 37 bored in the cooling chamber 34 along its periphery.

In the drawing with respect to this example, the planar electrode 33 of the bottom surface uses the whole surface as a working face. However, it is more preferable to provide an annular cooling surface formed of an insulating film on the outer peripheral part of the planar electrode 33 as described in Example 2.

The method of application of the electrode assembly of this invention will hereinafter be described.

As illustrated in FIGS. 10, 11 and 12, the terminal 5 of the electrode assembly 1 according to this invention is inserted in a holder 40 and connected to a high-frequency generator 41 of 60 W and 1 MHz through a switch 43. On the other hand, the inlet port 6 for the coolant is joined to a coolant tank 42 via a pump 44 through a pipe 48. Besides, the outlet port 7 is connected to the pipe 48 so as to circulate the coolant used for cooling through the coolant tank 42.

Ice is charged together with cooling water in the coolant tank 42, and the pump 44 is actuated to circulate the cooling water. When a distributed electrode 46 connected to the high-frequency generator 41 through a lead wire 45 is brought into contact with the human body on the opposite side to the affected part, and the switch 43 is turned on, a high-frequency current 49 radially flows through the human body 47. As a result, a surface of the skin coming into contact with the planar electrode 3, at which a current density is highest, and subcutaneous tissues thereabout are heated.

However, the planar electrode 3 of this invention is cooled from its back side by the cooling chamber 4. Therefore, as illustrated in FIG. 11, the surface 50 of the skin coming into contact with the planar electrode 3 and the tissue just under the surface 50 are neither heated nor coagulated, but only a subcutaneous tissue 51 at a distance of 2 mm or longer from the surface of the skin is heated, whereby a coagulated region 52 is formed.

With respect to this coagulated region 52, when the electrode assembly described in Example 1 is used, a coagulated region in a crescent form in section is formed, as shown in FIG. 11, because of the insufficient cooling capacity of the cooling chamber at its peripheral part. That may often bring about a result that an annular burn scar leaves.

When the electrode assembly described in Example 2 is used, the outer peripheral edge of the planar electrode 3 is thoroughly cooled as shown in FIG. 12 because the working face 10 of the planar electrode 3 is defined inside the outer periphery of the bottom surface of the cooling chamber 4. Therefore, the region positioned at the even depth from the surface of the skin is heated, thereby forming a coagulated region 52′ in a rectangular form in section.

An experiment on an animal flesh was practically carried out using an electrode assembly having the same structure as that of Example 2. The following result was obtained.

Using a planar electrode in a circular form, which has a working face of 14 mm in diameter $a$, a high-frequency current of 1 MHz and 60 W was caused to flow for 3 seconds.

A coagulated region 52′ of 14 mm in diameter and 6 mm in depth $b$ was formed at a distance $c$ of 2 mm from the surface of the skin. As shown in FIG. 12, this coagulated region 52′ was quite flat relative to the surface of the skin. No abnormality was recognized on the surface of the skin.

When a high-frequency current is caused to flow through the affected part from above the skin of the human body using a high-frequency heating and coagulating apparatus equipped with the electrode assembly according to the present invention, it is possible to selectively heat and coagulate the subcutaneous tissue at an even distance from the surface of the skin without being accompanied by any thermal injury or damage.

At that time, it is able to optionally control the depth, form and size of the coagulated region by changing the form and size of the working face of the planar electrode, and the intensity and application time of the high-frequency current.

Accordingly, the high-frequency heating and coagulating apparatus equipped with the electrode assembly can be used in remedy of the affected part of the human body, for example, superficial vascular diseases, i.e., hemangioma, lymphangioma, varix and tumors in a shallow site under the skin.

In addition, it can also be used in selectively atrophying the subcutaneous fat in order to remedy impairment in beauty due to uneven distribution of the subcutaneous fat, or loosened skins due to corpulence and/or ageing.

**Claims**

1.  An electrode assembly for a high-frequency heating and coagulating apparatus, comprising a planar electrode having a terminal, which is connected to a high-frequency power supply, and a working face in the form of a flat or convex surface, and a hollow cooling chamber for cooling the working face from the back side thereof, said planar electrode and hollow cooling chamber being formed integrally with each other.

2.  An electrode assembly as claimed in claim 1 for the high-frequency heating and coagulating apparatus, wherein the form of the planar electrode is circular, semicircular, crescent, oval or polygonal.

3.  An electrode assembly as claimed in one of claims 1 and 2 for the high-frequency heating and coagulating apparatus, wherein the cooling chamber is formed in such a manner that an annular cooling surface, which has been provided so as to surround the periphery of the working face of the planar electrode, is cooled from the back side thereof at the same time as the cooling of the working face of the planar electrode.

4.  An electrode assembly as claimed in one of claims 1 through 3 for the high-frequency heating and coagulating apparatus, wherein the planar electrode and hollow cooling chamber are made up integrally of an electrically conductive material and a peripheral portion of the planar electrode is coated annularly with an electrical insulating material having good thermal conductivity so that the coated portion and the region inside the coated portion function as an annular cooling surface and a working face of the planar electrode, respectively.

5.  An electrode assembly as claimed in one of claims 1 through 4 for the high-frequency heating and coagulating apparatus, wherein the planar electrode and hollow cooling chamber are formed integrally by a curved tube made of an electrically conductive material, an oval working face is formed in a convex surface of the tube, and the remaining portion of the tube is applied with a coating film of an electrical insulating material having good thermal conductivity to serve as a cooling surface.

6.  An electrode assembly as claimed in one of claims 1 through 3 for the high-frequency heating and coagulating apparatus, wherein the hollow cooling chamber is made up of an electrical insulating material having good thermal conductivity so as to have a bottom surface greater than a planar electrode, and the planar electrode is fitted in the bottom surface of the hollow cooling chamber at the substantial centre thereof, whereby a portion of the bottom surface, which is outside the periphery of the electrode, is taken as an annular cooling surface.

7.  An electrode assembly as claimed in one of claims 1 through 6 for the high-frequency heating and coagulating apparatus, wherein the hollow cooling chamber is provided with an inlet port and an outlet port for a coolant.

Fig.1

Fig.2

Fig.3

Fig.4

Fig.5

Fig.6

Fig.7

Fig.8

Fig.9

Fig.11

Fig.12

Fig.10